# FASCICULE DE BREVET EUROPEEN

(11) **EP 1 352 058 B1**
(45) Date de publication et mention de la délivrance du brevet: **10.05.2006**
(21) Numéro de dépôt: 01960864.5
(22) Date de dépôt: 03.08.2001
(51) Int. Cl.: C12N 11/10

(54) **PREPARATION DE MICRO-ORGANISMES ACCLIMATES IMMOBILISES, PROCEDE DE PRODUCTION ET APPLICATION A LA RELANCE DE FERMENTATIONS ARRETEES**
HERSTELLUNG VON IMMOBILISIERTEN AKKLIMATISIERTEN MIKROORGANISMEN, VERFAHREN ZU DEREN HERSTELLUNG UND DEREN VERWENDUNG ZUM ERNEUTEN STARTEN VON GESTOPTEN GÄRUNGEN
PREPARATION OF IMMOBILISED ACCLIMATED MICRO-ORGANISMS, PRODUCTION METHOD AND USE FOR REACTIVATING INTERRUPTED FERMENTATION PROCESSES

(30) Priorité: 04.08.2000 FR 0010319
(43) Date de publication de la demande: 15.10.2003
(73) Titulaire: Lallemand SAS, 31700 Blagnac (FR); Proenol-Industria, LDA, 4405-227 Canelas (PT)
(72) Inventeur: DULAU, Laurent, F-31000 Toulouse (FR); DE FATIMA TEIXEIRA CARDOSO DA SILVA, Maria, P-4405 Gulpilhares (PT); SANTOS, Leonor, P-5000 Vila Real (PT)
(74) Mandataire: Morelle, Guy Georges Alain
(86) Numéro de dépôt international: PCT/FR2001/002537
(87) Numéro de publication internationale: WO 2002/012473

(56) Documents cités:
- EP-A- 0 350 374
- WO-A-92/14544
- US-A- 4 380 552
- US-A- 5 070 019

## Description

La présente invention concerne une préparation de micro-organismes immobilisés dans des billes semi-humides capables d'assurer la reprise de fermentation de boissons fermentées.

Il est connu que les micro-organismes immobilisés peuvent servir à l'élaboration de boissons fermentées telles que le vin, la bière, le champagne, les boissons pétillantes à degré d'alcool variable.

Différents modes d'immobilisation de cellules sont connus, on peut citer en particulier :
- La demande de brevet EP 0350374 A1 qui décrit la préparation de microorganismes immobilisés dans des gels sensiblement déshydratés, dont l'utilisation est destinée à la préparation de boissons fermentées.
- La demande de brevet EP 0173915 B 1 qui décrit la préparation d'un biocatalyseur à cellules immobilisées dans un gel pour la fermentation et/ou la prise de mousse de vin champagnisé ou obtenu selon la méthode champenoise.
- Le brevet FR 2570959 qui décrit la méthode d'obtention de billes contenant des micro-organismes employés pour la fermentation des boissons citées plus haut.

Dans toutes ces techniques, les microorganismes sont immobilisés dans des matrices polymériques et introduits dans le moût dès le début du processus de fermentation, alors que les conditions du milieu sont favorables à leur activité métabolique. Cependant il arrive souvent que la fermentation soit ralentie voire stoppée avant que les teneurs en sucre et en alcool et/ou acide aient atteint les niveaux voulus. Il est alors nécessaire de relancer la fermentation par un nouvel apport de microorganismes.

Le problème posé actuellement est de disposer d'un outil permettant la relance d'une fermentation arrêtée sans étapes fastidieuses d'acclimatation des microorganismes. En effet, après arrêt accidentel d'une fermentation en cours, l'addition de microorganismes pour relancer la fermentation, par exemple de levure dans le cas des fermentations alcooliques, nécessite une étape dite d'acclimatation desdits microorganismes aux conditions du milieu de fermentation (en particulier du fait le la concentration d'alcool et/ou d'acide déjà produit); à défaut d'une telle étape, les microorganismes non acclimatés subissent un stress qui provoque généralement la perte de leur viabilité, et donc l'échec de la tentative de relance de fermentation. Par conséquent, les techniques actuellement utilisées doivent choisir entre deux schémas.

Un premier schéma fait appel à des microorganismes sous forme sèche, qui sont réhydratés puis progressivement acclimatés avant introduction dans le moût. L'acclimatation consiste à soumettre les microorganismes à une incubation pouvant durer de quelques heures à plusieurs jours, dans des milieux de concentration en alcool et/ou d'acidité croissante, et ce juste avant leur utilisation.

Un second schéma fait appel à des microorganismes préalablement acclimatés en dehors du lieu d'utilisation. Ils sont transportés sous forme humide et introduits tels quels dans le moût. Dans ce dernier cas, ils doivent être employés dans un délai bref de quelques jours, leur durée de conservation étant faible.

Cependant, il n'a pas été possible jusqu'à présent de combiner les avantages liés aux deux techniques: cellules séchées et cellules acclimatées. En effet, le séchage de microorganismes, qui permet généralement une bonne conservation et un transport facile, n'est pas applicable aux microorganismes acclimatés, levures notamment, car il provoque dans ce cas une mortalité importante et rapide.

La présente invention concerne une préparation de microorganismes, possédant à la fois les caractéristiques intéressantes des microorganismes sous forme séchée et des microorganismes acclimatés. En effet, il a été trouvé de façon surprenante que des microorganismes immobilisés et acclimatés dans des billes de polymères partiellement séchées permettait à la fois une bonne viabilité desdits microorganismes et le maintien de leur capacité à relancer une fermentation arrêtée, et ce même après un stockage prolongé de plusieurs mois.

Ainsi, la présente invention a pour objet des préparations de microorganismes, immobilisés dans des billes de polymère et acclimatés à l'alcool et/ou à l'acidité, aptes à assurer une relance des fermentations arrêtées. La présente invention a également pour objet un procédé d'obtention de telles préparations de microorganismes.

La préparation de microorganismes selon l'invention peut être réalisée avec n'importe quel genre de microorganisme vivant, susceptibles d'être immobilisé et acclimatés à l'alcool et/ou à l'acidité. De tels microorganismes sont décrits dans la littérature et sont bien connus de l'homme du métier. Selon un mode préféré de mise en oeuvre de la présente invention, les microorganismes immobilisés sont des levures, de préférence du genre Saccharomyces, telle que *Saccharomyces cerevisiae*.

Les billes dans lesquelles les microorganismes sont immobilisés sont composées de polymères réticulés formant une matrice, compatibles avec une bonne viabilité des microorganismes et avec leur utilisation dans des applications alimentaires. De tels polymères peuvent être des polysaccharides, de préférence un gel d'alginate de calcium. L'alginate est un polymère linéaire extrait d'algues et composé d'acide α-D-manuronique et α-L-guluronique. On peut également utiliser par exemple des matrices de polyacrylamide, de pectate ou de carraghénane.

On appelle "billes" des particules de forme sphérique ou de toute autre forme, obtenues par fractionnement de la matrice de polymère incluant les microorganismes. Selon une des caractéristiques de la présente invention, la taille moyenne de ces particules est comprise entre 0,1 et 5 mm, de préférence entre 1 et 3 mm. Selon une autre caractéristique de l'invention, la proportion de microorganismes par rapport au polymère est comprise entre 1 et 50%, de préférence entre 5 et 20% en poids sec.

Selon une autre caractéristique de l'invention, l'humidité des billes polymériques est contrôlées de telle sorte que celles-ci présentent un aspect sec, mais contiennent un proportion d'eau suffisante pour maintenir la viabilité des microorganismes. L'humidité est déterminée par la mesure de l'activité de l'eau Aw dans les billes, ladite activité étant comprise entre 0,1 et 0,5, et de préférence entre 0,3 et 0,4.

Selon une caractéristique de l'invention les microorganismes vivants immobilisés sont également acclimatés c'est-à-dire qu'ils présentent un état physiologique leur permettant de développer un métabolisme fermentatif dès leur introduction dans des moûts à des concentrations en alcool et/ou en acide élevées, sans chute importante d'activité. En d'autres termes, les microorganismes vivants, acclimatés et immobilisés de la préparation selon l'invention présentent la capacité de démarrer directement après une étape de réhydratation, une fermentation dans un moût présentant un degré alcoolique compris entre 5 et 15° et un pH compris entre 2,8 et 4,0.

En outre, les microorganismes vivants, acclimatés et immobilisés de la préparation selon l'invention conservent ces propriétés durant plusieurs mois, ce qui présente un avantage décisif pour leur exploitation commerciale. Pour cela, il suffit de conserver les billes contenant les micro-organismes partiellement déshydratés, dans un emballage étanche à la vapeur d'eau, maintenu de préférence à une température relativement basse, classiquement d'environ 4°C. Dans ces conditions on obtient une excellente conservation des microorganismes pendant au moins six mois de stockage.

La présente invention a également pour objet un procédé d'obtention d'une préparation de microorganismes vivants, acclimatés à l'alcool et/ou à l'acidité, immobilisés dans des billes de polymère partiellement déshydratées, telle que décrite ci-avant, ledit procédé consistant à réaliser les opérations suivantes :
a)- acclimater lesdits microorganismes à l'alcool et / ou à l'acide,
b)- immobiliser lesdits microorganismes dans des billes de polymère,
c)- déshydrater partiellement lesdites billes,
dans l'ordre chronologique [a) puis b) puis c)], ou bien [b) puis a) puis c)].

Selon la présente invention, les étapes d'acclimatation et d'immobilisation peuvent être effectuées dans un ordre indifférent, mais doivent obligatoirement précéder l'étape de déshydratation partielle.

Selon un mode préféré de réalisation de la présente invention, les microorganismes ainsi préparés sont des levures, de préférence du genre Saccharomyces, qui sont immobilisées dans des billes de polysaccharides réticulés, de préférence dans un gel d'alginate, dans une proportion de 1 à 50%, de préférence de 5 à 20% en poids sec, par rapport audit polymère.

On réalise au départ une culture de microorganismes dans un milieu de culture classique. Puis à partir de ces microorganismes, on réalise soit d'abord l'acclimatation, soit d'abord l'immobilisation des microorganismes, les techniques utilisées pour chaque opération étant indépendantes l'une de l'autre ainsi que de l'ordre choisi pour les exécuter.

Il est possible, dans une variante de mise en oeuvre, de ne pas utiliser immédiatement les microorganismes obtenus par culture, mais de les conserver sous forme séchés pour la durée désirée. Dans ce cas, on prendra soin de les réhydrater selon une des méthodes bien connues des professionnels, avant de réaliser soit l'acclimatation, soit l'immobilisation des microorganismes.

En ce qui concerne l'immobilisation de microorganismes, les techniques en sont connues. Par exemple, les procédés décrits dans les brevets cités précédemment sont utilisables. En général, on réalise la réticulation d'une solution polymérique contenant les microorganismes à enrober, puis on fractionne de la matrice obtenue par des moyens mécaniques, le plus souvent par broyage, pour former des billes de polymère contenant les microorganismes ainsi piégés. Une autre technique couramment employé consiste à faire passer les microorganismes en suspension dans une solution polymérique, à travers une buse pour former des gouttes, qui sont mises en contact avec un agent gélifiant dans une phase hydrophobe sous agitation. Selon un aspect particulier de l'invention, les microorganismes sont immobilisés dans une matrice de polymère capable de former un gel insoluble dans le vin, tel que l'alginate, selon un procédé connu (comme par exemple celui décrit par DIVIES *et al*, in Proc. Colloque SFR, Compiègne, 1979).

En ce qui concerne l'acclimatation, les techniques en sont également bien connues. En général, on incube des microorganismes séchés dans une solution de degré l'alcool ou d'acidité comparable à celui du milieu dans lequel on souhaite les faire agir. Par exemple, dans le cas de levures destinées à la relance de la fermentation d'un milieu alcoolique, on incube les levures sèches pendant 30 minutes à 37°C dans un vin titrant 10° d'alcool additionné de saccharose. Puis, on récupère les levures par exemple par centrifugation, et on les met en suspension dans une solution d'alginate en vue de l'étape d'immobilisation.

Si les levures sont déjà sous forme immobilisées, on réalise une réhydratation puis une incubation comme précédemment. On termine par l'étape de séchage.

L'étape de déshydratation partielle peut être effectuée par une des techniques suivantes: lyophilisation, séchage sur lit fluidisé, étuvage, jusqu'à obtention d'une activité de l'eau Aw dans lesdites billes de polymère, comprise entre 0,1 et 0,5, de préférence entre 0,3 et 0,4. Ces techniques sont choisies du fait qu'elles permettent de contrôler le taux d'humidité atteint, c'est-à-dire de réaliser une déshydratation ménagée. On obtient des billes d'aspect sec, mais contenant une petite proportion d'humidité de manière à maintenir la viabilité des microorganismes.

Selon une modalité particulière de mise en oeuvre de l'invention, on conserve les billes partiellement déshydratées contenant les micro-organismes acclimatés, dans un emballage étanche à la vapeur d'eau, qui est de préférence maintenu à une température relativement basse, de préférence d'environ 4°C. Dans ces conditions on obtient une excellente conservation des microorganismes pendant plusieurs mois de stockage.

Ainsi préparées et éventuellement stockées, les préparations de microorganismes selon l'invention peuvent être utilisées, immédiatement ou dans un délai de plusieurs mois, directement après une simple réhydratation, et sans étape d'acclimatation, à la relance de fermentations arrêtées, en particulier à la relance des fermentations alcooliques arrêtées, telles que la fermentation des vins rouges ou blancs, de l'hydromel, ou de toute autre boisson préparée par fermentation alcoolique.

D'autres caractéristiques et avantages de l'invention apparaîtront à la lumière des exemples non limitatifs ci-après.

### EXEMPLES

### Exemple 1: Préparation de billes d'alginate semi-humides contenant des levures Saccharomyces cerevisiae immobilisées, préalablement acclimatées à l'alcool

Dans cet exemple, des levures préalablement acclimatées à l'alcool, sont immobilisées dans des billes préparées à partir d'une solution d'alginate de sodium. La souche de levure *Saccharomyces cerevisiae* L43^{(R)} commercialisée par la société Lallemand (France) est utilisée. L'étape d'acclimatation consiste à incuber pendant 30 minutes à 37°C les levures à raison de 100 g de levures sèches pour 1 litre de liquide, dans un vin titrant 10° d'alcool et additionné de 100 g de saccharose par litre. Après incubation, les levures sont récupérées par centrifugation et resuspendues dans la solution d'alginate de sodium à 4%.
Cette solution passe ensuite dans un système de tubes soumis à des vibrations qui permettent la formation de gouttes. Ces gouttes sont ensuite gélifiées au contact d'une solution 0,2 M de chlorure de calcium. Le temps de contact est de 30 minutes.

Les billes ainsi formées sont lavées par immersion pendant 10 minutes dans de l'eau désionisée. Les billes sont ensuite séchées partiellement sur lit fluidisé jusqu'à obtenir une activité en eau Aw comprise entre 0.3 et 0.4. La température de séchage est inférieure ou égale à 40°C. Le diamètre des billes obtenues est de 2 à 4 mm. Après les contrôles qualité les levures sont emballées et stockées à 4°C avant utilisation.

### Exemple 2 : Acclimatation de levures préalablement immobilisées

Des levures obtenues par réhydratation dans l'eau de levures sèches actives L2226^{(R)} commercialisée par la société Lallemand (France), sont immobilisées puis acclimatées. Des billes d'alginate sont préparées comme dans l'exemple 1 ci-dessus avec des levures non acclimatées. Les billes obtenues sont ensuite récupérées par tamisage et incubées 30 minutes à 37°C dans un vin à 10° d'alcool additionné de 100 g de saccharose par litre. Après tamisage et lavage par une solution de chlorure de sodium à 9 g/l, les billes sont soumise à l'étape de séchage dans les conditions décrites dans l'exemple 1.
Après les contrôles qualité les levures sont emballées et stockées à 4°C avant utilisation.

### Exemple 3: Stabilité des levures au cours du stockage

Afin d'estimer la stabilité des levures, l'activité de billes contenant la levure *Saccharomyces cerevisiae* est mesurée au temps 0 (juste après la production des billes) et après 6 mois de conservation à 4°C. Le contrôle suivant est réalisé :
- 30 grammes de billes préparées selon l'exemple 1 ci-dessus sont placées dans une solution de glucose à 50 g/l (volume 100 ml) à 37°C.
- La solution est maintenue à 37°C et l'évolution de la concentration du sucre est suivie au cours du temps.

Dans les deux échantillons (t = 0 et t = 6 mois), une concentration en sucre inférieure à 2 g/l correspondant à la fin de la fermentation est obtenue au bout de 2 heures. La fin de la fermentation est obtenue après le même temps d'incubation. Les levures immobilisées préparées selon l'exemple 1 sont donc stables pendant au moins 6 mois de stockage à 4°C.

### Exemple 4: Application au vin blanc

Le vin blanc utilisé dans cet exemple est un vin de Sauvignon présentant les caractéristiques suivantes :
- degré d'alcool acquis au moment de l'inoculation avec les billes : 12,75 °
- sucres résiduels : 14,64 g/l

On procède à la reprise de fermentation en utilisant la levure L43^{(R)} commercialisée par la société Lallemand (France), immobilisée sous forme de billes obtenues selon l'exemple 1, conditionnée en unités de 5 kg. La dose d'utilisation est de 200 g de billes par hl de vin. Pour les besoins de l'essai, le vin en arrêt de fermentation est réparti en deux bonbonnes de 25 1 (modalités 1 et 2) et deux cuves de 150 hl (modalités 3 et 4).

Ces modalités sont les suivantes (la température est régulée entre 18 et 20°C) :
- modalité 1. témoin négatif: vin non ensemencé
- modalité 2. témoin positif: vin ensemencé par des levures L43^{(R)}, sous forme de levure sèche, à la dose de 20 g/hl, réhydratées, non acclimatées
- modalité 3. vin ensemencé avec des levures L43 (R), sous forme de levure sèche, à la dose de 20 g/hl, réhydratées, acclimatées à l'alcool selon le protocole dit I.T.V. connu par l'homme de l'art (durée des phases de réhydratation et d'acclimatation : 7 jours)
- modalité 4. vin ensemencé avec des levures L43^{(R)} immobilisées obtenues selon l'exemple 1, préparées selon le protocole suivant:

Préparation des levures L43^{(R)} immobilisées (modalité 4) :
a) 30 kg de billes en sac sont plongées, à la température de 37°C, dans une cuve contenant une solution de 60 l d'eau contenant 600 g de glucose / fructose (50/50) et 480 g de NaCl.
b) Après 30 minutes, les sacs sont retirés et trempés, dans les mêmes conditions, dans une nouvelle solution, identique à la précédente.
c) Ensuite les sacs sont immergés dans la cuve contenant le vin dont la fermentation alcoolique doit être relancée.

Pour chacune des 4 modalités, on suit l'évolution du taux de sucre en fonction du temps. Les résultats de ce suivi sont présentés dans le tableau 1 suivant :

**TABLEAU 1**

| | | | | |
|---|---|---|---|---|
| JOURS / | | SUCRES RÉSIDUELS (g/l) | | |
| | Modalité 1 | Modalité 2 | Modalité 3 | Modalité 4 |
| 0 | 14,64 | 14,64 | 14,64 | 14,64 |
| 1 | | | | 13,66 |
| 4 | | | | 12,50 |
| 5 | | | | 12,10 |
| 7 | 14,64 | 14,67 | 14,64 | |
| 9 | | | 12,46 | |
| 10 | | | | 8,88 |
| 11 | | | 10,54 | 8,00 |
| 14 | 16,62 | 14,64 | 8,66 | 5,71 |
| 17 | | | | 4,34 |
| 20 | | | 5,55 | 2,48 |
| 22 | 14,65 | 14,60 | 3,07 | |
| 24 | | | | 1,80 |
| 26 | 14,64 | 14,62 | 2,00 | |

On constate que le procédé selon la présente invention (modalité 4) permet d'obtenir un achèvement de la fermentation, au bout d'une durée plus courte que celle observée avec les mêmes levures acclimatées selon la méthode habituelle (modalité 3), toutes choses étant égales par ailleurs. On n'observe pas, dans l'un et l'autre cas, d'augmentation de l'acidité volatile.
Le procédé d'application selon la présente invention présente ainsi deux avantages essentiels:
- il permet une économie de temps de 2 jours pour la durée totale de l'opération de fermentation,
- il demande 1 heure et non plus 7 jours de préparation et de suivi du pied de cuve.

### Exemple 5: Application au vin rouge

Le vin rouge utilisé dans cet exemple est un vin de cépage Merlot présentant les caractéristiques suivantes :
- degré d'alcool acquis au moment de l'inoculation avec les billes : 99,9 g éthanol/l
- pH : 3,2
- sucres résiduels : 19,29 g/l

On procède à la reprise de fermentation en utilisant la levure L2226^{(R)} commercialisée par la société Lallemand (France), immobilisée sous forme de billes obtenues selon l'exemple 2, conditionnée en unités de 5 kg. La dose d'utilisation est de 200 g de billes par hl de vin. Pour les besoins de l'essai, le vin en arrêt de fermentation est réparti en deux bonbonnes de 25 1 (modalités 1 et 2) et deux cuves de 5 hl (modalités 3 et 4).

Ces modalités sont les suivantes (la température est régulée entre 20 et 25°C) :
- modalité 1. témoin négatif : vin non ensemencé,
- modalité 2. témoin positif : vin ensemencé par des levures L2226^{(R)}, sous forme de levure sèche, à la dose de 20 g/hl, réhydratées, non acclimatées,
- modalité 3. vin ensemencé avec des levures L2226^{(R)}, sous forme de levure sèche, à la dose de 20 g/hl, réhydratées, acclimatées à l'alcool selon le protocole I.T.V. connu par l'homme de l'art (durée des phases de réhydratation et d'acclimatation : 6 jours),
- modalité 4. vin ensemencé avec des levures L2226^{(R)} immobilisées, obtenues selon l'exemple 2, et préparées selon le protocole décrit ci-après :.

Préparation des levures immobilisées (modalité 4):
a) 1 kg de billes en sacs sont plongées, à la température de 37°C, à une solution de 21 d'eau contenant 20 g de glucose / fructose (50/50) et 16 g de NaCl.
b) Après 30 minutes, les sacs sont retirés et trempés, dans les mêmes conditions dans une nouvelle solution identique à la précédente.
c) Ensuite les sacs sont immergés dans la cuve contenant le vin dont la fermentation alcoolique doit être relancée.

Pour chaque modalité, on suit, l'évolution du taux de sucre en fonction du temps. Les résultats de ce suivi sont présentés dans le tableau 2 suivant :

**TABLEAU 2**

| JOURS / | Modalité 1 | Modalité 2 | Modalité 3 | Modalité 4 |
|---|---|---|---|---|
| | sucres résiduels (g/l) / éthanol (g/l) | sucres résiduels (g/l) / éthanol (g/l) | sucres résiduels (g/l) / éthanol (g/l) | sucres résiduels (g/l) / éthanol (g/l) |
| 0 | 19,3 / 99,9 | 19,3 / 99,9 | 19,30 / 99,90 | 19,30 / 99,90 |
| 2 | Inchangés | Inchangés | Inchangés | 18,87 / 101,48 |
| 4 | Inchangés | Inchangés | Inchangés | 16,90 / 101,93 |
| 5 | Inchangés | Inchangés | Inchangés | 14,38 / 102,36 |
| 6 | Inchangés | Inchangés | Inchangés | 13,73 / 102,37 |
| 7 | Inchangés | Inchangés | 19,29 / 100,66 | - |
| 11 | Inchangés | Inchangés | 16,93 / 101,35 | 4,20 / 104,86 |
| 12 | Inchangés | Inchangés | 11,63 / 102,41 | 2,76 / 105,29 |
| 13 | Inchangés | Inchangés | 7,46 / 103,53 | 2,21 / 105,31 |
| 14 | Inchangés | Inchangés | 3,81 / 104,91 | - |
| 15 | Inchangés | Inchangés | 1,83 / 105,39 | - |

On constate que le procédé selon la présente invention (modalité 4) permet d'obtenir un achèvement de la fermentation, au bout dune durée inférieure à celle observée avec les mêmes levures acclimatées selon la méthode habituelle (modalité 3), toutes choses étant égales par ailleurs. On n'observe pas, dans l'un et l'autre cas, de montée de l'acidité volatile.

Le procédé d'application selon la présente invention présente ainsi deux avantages essentiels:
1. il permet un gain de 2 jours pour la durée totale de l'opération de fermentation,
2. il demande 1 heure au lieu de 6 jours de préparation et de suivi du pied de cuve.

### Exemple 6 : Application à l'hydromel

L'hydromel utilisé dans cet exemple est issu d'un mélange 300 g de miel / 1 litre d'eau, le but étant de fabriquer de l'hydromel doux titrant 12,5 % d'alcool. Au moment de l'arrêt de fermentation, il possédait les caractéristiques suivantes :
- degré d'alcool acquis au moment de l'inoculation avec les billes : 10,5 % d'alcool
- sucres résiduels totaux : 87,5 g/l
- sucres résiduels à fermenter : 34 g/l
   On procède à la reprise de fermentation en utilisant la levure EC1118^{(R)} commercialisée par la société Lallemand (France), immobilisée dans des billes d'alginate de calcium, obtenues selon l'exemple 1, conditionnées en unités de 200 g. La dose d'utilisation est de 200 g de billes pour 100 l d'hydromel.

Pour les besoins de l'essai, l'hydromel en arrêt de fermentation est réparti en deux bonbonnes de 50 1 (modalités 1 et 2). Ces modalités sont les suivantes (la température est régulée entre 20 et 25°C) :
- modalité 1. hydromel ensemencé avec la levure EC1118^{(R)} immobilisées et préparées selon le protocole décrit ci-après:
   a) un sac de 200 g de billes est plongé, à la température de 37°C, dans une solution de 400 ml d'eau contenant 4 g de glucose / fructose (50/50) et 3,2 g de NaCl.
   b) puis après 30 minutes, le sac est retiré et trempé, dans les mêmes conditions dans une nouvelle solution identique à la précédente.
   c) ensuite le sac est immergé dans la cuve contenant l'hydromel dont la fermentation alcoolique doit être relancée.
- modalité 2. hydromel ensemencé avec la levure EC1118^{(R)}, sous forme de levures sèches, à la dose de 20 g/hl, réhydratées, acclimatées à l'alcool selon le protocole I.T.V. adapté à l'hydromel (durée des phases de réhydratation et d'acclimatation : 4 jours).

On suit, pour chaque modalité, l'évolution du taux de sucre en fonction du temps. Les résultats de ce suivi sont présentés dans le tableau 3 suivant :

**TABLEAU 3**

| JOURS / | SUCRES RÉSIDUELS (g/l) | |
|---|---|---|
| | Modalité 1 | Modalité 2 |
| 0 | 87,50 | 87,50 |
| 1 | 86,00 | 85,00 |
| 4 | 75,00 | 72,50 |
| 5 | 71,50 | 68,50 |
| 7 | 66,20 | 64,00 |
| 9 | 58,50 | 59,50 |
| 11 | 50,00 | 54,70 |
| 14 | 36,70 | 46,00 |
| 20 | 36,50 | 36,20 |

On constate que le procédé selon la présente invention (modalité 1) permet d'obtenir un achèvement de la fermentation au bout d'une durée inférieure à celle observée avec les mêmes levures acclimatées selon la méthode habituelle (modalité 2), toutes choses étant égales par ailleurs.

Le procédé d'application de la présente invention présente ainsi deux avantages essentiels:
1. il permet un gain de presque 6 jours pour la durée totale de l'opération de fermentation;
2. il demande 1 heure au lieu de 4 jours de préparation et de suivi du pied de cuve.

## Revendications

1. Préparation de microorganismes vivants, acclimatés à l'alcool et/ou à l'acidité, immobilisés dans des billes de polymère partiellement déshydratées.

2. Préparation selon la revendication 1, ***caractérisée en ce que*** lesdits microorganismes vivants, acclimatés et immobilisés, présentent la capacité de démarrer directement après une étape de réhydratation, une fermentation dans un moût présentant un degré alcoolique compris entre 5 et 15° et un pH compris entre 2,8 et 4,0.

3. Préparation selon la revendication 2, ***caractérisée en ce que*** lesdits microorganismes vivants, acclimatés et immobilisés, présentent après une période de conservation d'une durée de 1 jour à au moins six mois, la capacité de démarrer directement après une étape de réhydratation, une fermentation dans un moût présentant un degré alcoolique compris entre 5 et 15° et un pH compris entre 2,8 et 4,0,.

4. Préparation selon la revendication 1, ***caractérisée en ce que*** lesdits microorganismes sont des levures, de préférence du genre Saccharomyces.

5. Préparation selon la revendication 1, ***caractérisée en ce que*** lesdites billes de polymère sont composées de polysaccharides réticulés, de préférence d'un gel d'alginate.

6. Préparation selon la revendication 1, ***caractérisée en ce que*** dans lesdites billes de polymère, l'activité de l'eau est comprise entre 0,1 et 0,5, de préférence entre 0,3 et 0,4.

7. Préparation selon la revendication 1, ***caractérisée en ce que*** lesdites billes ont une taille comprise entre 0,1 et 5 mm, de préférence entre 1 et 3 mm.

8. Préparation selon la revendication 1, ***caractérisée en ce que*** lesdites billes contiennent lesdits microorganismes dans une proportion de 1 à 50%, de préférence de 5 à 20% en poids sec, par rapport audit polymère.

9. Procédé d'obtention d'une préparation de microorganismes vivants, acclimatés à l'alcool et/ou à l'acidité, immobilisés dans des billes de polymère partiellement déshydratées, ledit procédé consistant à réaliser les opérations suivantes :
a)- acclimater lesdits microorganismes à l'alcool et / ou à l'acide,
b)- immobiliser lesdits microorganismes dans des billes de polymère,
c)- déshydrater partiellement lesdites billes,
dans l'ordre chronologique [a) puis b) puis c)], ou bien [b) puis a) puis c)].

10. Procédé selon la revendication 9, dans lequel lesdits microorganismes sont des levures, de préférence du genre Saccharomyces.

11. Procédé selon la revendication 9, dans lequel lesdites billes de polymère sont composées de polysaccharides réticulés, de préférence d'un gel d'alginate.

12. Procédé selon la revendication 9, dans lequel lesdits microorganismes immobilisés dans lesdites billes de polymère sont dans une proportion de 1 à 50%, de préférence de 5 à 20% en poids sec, par rapport audit polymère.

13. Procédé selon la revendication 9, dans lequel ladite déshydratation partielle est effectuée par une des techniques suivantes: lyophilisation, séchage sur lit fluidisé, étuvage, jusqu'à obtention d'une activité de l'eau dans lesdites billes de polymère, comprise entre 0,1 et 0,5, de préférence entre 0,3 et 0,4.

14. Application d'une préparation de microorganismes selon l'une des revendications 1 à 8, à la reprise de fermentations arrêtées.

15. Application d'une préparation de microorganismes selon l'une des revendications 1 à 8, à la reprise d'une fermentation alcoolique arrêtée.

## Patentansprüche

1. Zubereitung von lebenden Mikroorganismen, die an Alkohol und/oder an Acidität akklimatisiert sind und in teilweise dehydrierten Polymerkugeln immobilisiert sind.

2. Zubereitung nach Anspruch 1, **dadurch gekennzeichnet, dass** die akklimatisierten und immobilisierten lebenden Mikroorganismen die Fähigkeit aufweisen, direkt nach einem Rehydrierungsschritt eine Gärung in einer Maische zu starten, die einen Alkoholgrad zwischen 5 und 15° und einen pH zwischen 2,8 und 4,0 aufweist.

3. Zubereitung nach Anspruch 2, **dadurch gekennzeichnet, dass** die akklimatisierten und immobilisierten lebenden Mikroorganismen nach einer Aufbewahrungszeit mit einer Dauer von einem Tag bis zu mindestens sechs Monaten die Fähigkeit aufweisen, direkt nach einem Rehydrierungsschritt eine Gärung in einer Maische zu starten, die einen Alkoholgrad zwischen 5 und 15° und einen pH zwischen 2,8 und 4,0 aufweist.

4. Zubereitung nach Anspruch 1, **dadurch gekennzeichnet, dass** die Mikroorganismen Hefepilze sind, vorzugsweise von der Gattung Saccharomyces.

5. Zubereitung nach Anspruch 1, **dadurch gekennzeichnet, dass** die Polymerkugeln aus vernetzten Polysacchariden bestehen, vorzugsweise aus einem Alginatgel.

6. Zubereitung nach Anspruch 1, **dadurch gekennzeichnet, dass** die Wasseraktivität in den Polymerkugeln zwischen 0,1 und 0,5, vorzugsweise zwischen 0,3 und 0,4, beträgt.

7. Zubereitung nach Anspruch 1, **dadurch gekennzeichnet, dass** die Kugeln eine Größe zwischen 0,1 und 5 mm, vorzugsweise zwischen 1 und 3 mm, aufweisen.

8. Zubereitung nach Anspruch 1, **dadurch gekennzeichnet, dass** die Kugeln Mikroorganismen in einem Verhältnis von 1 bis 50 %, vorzugsweise von 5 bis 20 % im Trockengewicht, in Bezug auf das Polymer enthalten.

9. Verfahren zur Herstellung einer Zubereitung von lebenden Mikroorganismen, die an Alkohol und/oder an Acidität akklimatisiert sind und in teilweise dehydrierten Polymerkugeln immobilisiert sind, wobei das Verfahren darin besteht, die folgenden Vorgänge auszuführen:
a) Akklimatisieren der Mikroorganismen an den Alkohol und/oder an die Säure,
b) Immobilisieren der Mikroorganismen in Polymerkugeln,
c) teilweises Dehydrieren der Kugeln,
und zwar in der chronologischen Reihenfolge [a) dann b) dann c)] oder [b) dann a) dann c)].

10. Verfahren nach Anspruch 9, wobei die Mikroorganismen Hefepilze sind, vorzugsweise von der Gattung Saccharomyces.

11. Verfahren nach Anspruch 9, wobei die Polymerkugeln aus vernetzten Polysacchariden bestehen, vorzugsweise aus einem Alginatgel.

12. Verfahren nach Anspruch 9, wobei die in den Polymerkugeln immobilisierten Mikroorganismen in einem Verhältnis von 1 bis 50 %, vorzugsweise von 5 bis 20 % im Trockengewicht, in Bezug auf das Polymer vorhanden sind.

13. Verfahren nach Anspruch 9, wobei die teilweise Dehydrierung durch eine der folgenden Techniken ausgeführt wird: Gefriertrocknung, Trocknung auf einem Wirbelbett, Ofentrocknung, und zwar bis zur Erzielung einer Wasseraktivität in den Polymerkugeln zwischen 0,1 und 0,5, vorzugsweise zwischen 0,3 und 0,4.

14. Verwendung einer Mikroorganismen-Zubereitung nach einem der Ansprüche 1 bis 8 zur Wiederaufnahme von zum Stillstand gekommenen Gärungen.

15. Verwendung einer Mikroorganismen-Zubereitung nach einem der Ansprüche 1 bis 8 zur Wiederaufnahme einer zum Stillstand gekommenen alkoholischen Gärung.

## Claims

1. Preparation of living micro-organisms, which are acclimatised to alcohol and/or to acidity and immobilised in partially dehydrated polymer beads.

2. Preparation according to claim 1, ***characterised in that*** said living micro-organisms, acclimatised and immobilised, have the ability to start fermenting, directly after a rehydration step, in a must which has an alcoholic degree of between 5 and 15° and a pH of between 2.8 and 4.0.

3. Preparation according to claim 2, ***characterised in that*** said living micro-organisms, acclimatised and immobilised, have the ability to start fermenting, after a preservation period lasting between 1 day and at least six months, directly after a rehydration step, in a must which has an alcoholic degree of between 5 and 15° and a pH of between 2.8 and 4.0

4. Preparation according to claim 1, ***characterised in that*** said micro-organisms are yeasts, preferably of the Saccharomyces genus.

5. Preparation according to claim 1, ***characterised in that*** said polymer beads are made up of reticulated polysaccharides, preferably of an alginate gel.

6. Preparation according to claim 1, ***characterised in that,*** in said polymer beads, the activity of the water is between 0.1 and 0.5, preferably between 0.3 and 0.4.

7. Preparation according to claim 1, ***characterised in that*** the size of said beads is between 0.1 and 5 mm, preferably between 1 and 3 mm.

8. Preparation according to claim 1, ***characterised in that*** said beads contain said micro-organisms in a proportion of between 1 and 50 %, preferably between 5 and 20 % dry weight, relative to said polymer.

9. Method of obtaining a preparation of micro-organisms which are acclimatised to alcohol and/or to acidity and immobilised in partially dehydrated polymer beads, said method comprising the following operations:
a)- acclimatising said micro-organisms to alcohol and/or to acid,
b)- immobilising said micro-organisms in polymer beads, and
c)- partially dehydrating said beads,
which operations are carried out in the chronological order [a) then b) then c)], or indeed [b) then a) then c)].

10. Method according to claim 9, in which said micro-organisms are yeasts, preferably of the Saccharomyces genus.

11. Method according to claim 9, in which said polymer beads are made up of reticulated polysaccharides, preferably of an alginate gel.

12. Method according to claim 9, in which said micro-organisms, immobilised in said polymer beads, are in a proportion of between 1 and 50 %, preferably between 5 and 20 % dry weight, relative to said polymer.

13. Method according to claim 9, in which said partial dehydration is effected by one of the following techniques: lyophilisation, drying on a fluidised bed, using a drying cupboard, until an activity of the water of between 0.1 and 0.5, preferably between 0.3 and 0.4, is obtained in said polymer beads.

14. Use of a preparation of micro-organisms according to one of claims 1 to 8 for the re-starting of interrupted fermentation processes.

15. Use of a preparation of micro-organisms according to one of claims 1 to 8 for the re-starting of an interrupted alcoholic fermentation.
